(19) Europäisches Patentamt
European Patent Office
Office européen des brevets

(11) EP 1 017 801 B1

(12) **EUROPEAN PATENT SPECIFICATION**

(45) Date of publication and mention of the grant of the patent:
**16.08.2006 Bulletin 2006/33**

(21) Application number: **98944189.4**

(22) Date of filing: **23.09.1998**

(51) Int Cl.:
***C12N 15/11*** *(2006.01)* ***A61K 47/26*** *(2006.01)*
***C07H 21/04*** *(2006.01)*

(86) International application number:
**PCT/IE1998/000080**

(87) International publication number:
**WO 1999/015649 (01.04.1999 Gazette 1999/13)**

# (54) COMPOSITION AND METHOD FOR ENHANCING PARACELLULAR TRANSPORT ACROSS CELL LAYERS

ZUSAMMENSETZUNGEN UND VERFAHREN ZUR ERHÖHUNG DES PARAZELLULÄREN TRANSPORTES DURCH ZELLSCHICHTEN

COMPOSITION ET PROCEDE D'AMELIORATION DU TRANSPORT PARACELLULAIRE A TRAVERS LES COUCHES CELLULAIRES

(84) Designated Contracting States:
**AT BE CH CY DE DK ES FI FR GB GR IE IT LI LU MC NL PT SE**

(30) Priority: **24.09.1997 US 59644 P**
**10.11.1997 IE 970794**

(43) Date of publication of application:
**12.07.2000 Bulletin 2000/28**

(73) Proprietor: **Merrion Research I Limited**
**Dublin 24 (IE)**

(72) Inventors:
- **O'MAHONY, Daniel Joseph**
**Blackrock,**
**County Dublin (IE)**
- **CAGNEY, Gerard**
**Blackrock,**
**County Dublin (IE)**

(74) Representative: **Ryan, Anne Mary et al**
**c/o Anne Ryan & Co.**
**60 Northumberland Road**
**Ballsbridge**
**Dublin 4 (IE)**

(56) References cited:
**WO-A-97/32982**

- **ANDO-AKATSUKA, Y. ET AL.: "Interspecies diversity of the occludin sequence: cDNA cloning of human, mouse, dog, and rat-kangaroo homologues." THE JOURNAL OF CELL BIOLOGY., vol. 133, April 1996, pages 43-47, XP002095629 cited in the application**
- **WONG, V. & GUMBINER, B.: "A synthetic peptide corresponding to the extracellular domain of occludin perturbs the tight junction permeability barrier" THE JOURNAL OF CELL BIOLOGY., vol. 136, 27 January 1997, pages 399-409, XP002095630**
- **FURUSE M ET AL: "OCCLUDIN: A NOVEL INTEGRAL MEMBRANE PROTEIN LOCALIZING AT TIGHT JUNCTIONS" JOURNAL OF CELL BIOLOGY, vol. 123, no. 6, 1993, pages 1777-1788, XP002060057 cited in the application**
- **HOCHMAN J ET AL: "MECHANISMS OF ABSORPTION ENHANCEMENT AND TIGHT JUNCTION REGULATION" JOURNAL OF CONTROLLED RELEASE, vol. 29, 1994, pages 253-267, XP002054267**
- **KEVIL C G ET AL: "Expression of zonula occludens and adherens junctional proteins in human venous and arterial endothelial cells: role of occludin in endothelial solute barriers." MICROCIRCULATION, (1998) 5 (2-3) 197-210., XP002095631**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

Technical Field

The present invention relates to a composition and method for enhancing paracellular transport across cell layers in an animal.

Background Art

Transepithelial and transendothelial transport relate to the movement of solutes across a cell layer. In transcellular transport, solutes move both through and between cells. Movement of solutes through cells, for example across the lumenal and basolateral membranes of epithelial cells, requires transcellular transport. Transcellular transport may be active or passive depending on the solute in question. Active transcellular transport is carrier mediated and energy dependent, and permits solutes to move against their electrochemical gradients. Passive transcellular transport depends on electrochemical gradients generated by active transcellular transport and on the permeability of the cell membrane to the solute.

Movement of solutes between cells, through the tight junctions which bind cells together into a layer as with the epithelial cells of the gastro-intestinal tract, is termed paracellular transport. Paracellular transport is passive. Paracellular transport depends on electrochemical gradients generated by transcellular transport and on solvent drag through tight junctions. Tight junctions form an intercellular barrier which separates the apical and basolateral fluid compartments of a cell layer. Movement of a solute through a tight junction from apical to basolateral compartments depends on the "tightness" of the tight junction for that solute.

The "tightness" of tight junctions varies among different epithelial cell layers. For example, in the gastrointestinal tract of the human, tight junctions in the colon are tighter than those in the ileum. In the kidney of the human, tight junctions in the ascending limb of the Loop of Henle are tighter than those in the proximal tubule. The "tightness" of tight junctions also varies among different endothelial cell layers. For example, in the gastrointestinal tract, capillary endothelial cells have irregular tight junctions between adjacent cells enabling passage of some solutes between the cells. In the brain and spinal cord of the human, capillary endothelial cells have virtually continuous tight junctions between the adjacent cells, almost completely preventing passage of solutes between the cells.

Epithelial cell layers and endothelial cell layers present a significant barrier to the movement of solutes across cell layers. This can result in significant problems or barriers for drug absorption regardless of the route of administration of the drug. Therefore, new strategies for delivering drugs across epithelial and endothelial cell layers are needed.

Occludin is a -65kD integral membrane protein which is localized at tight junctions in epithelial cell layers and in endothelial cell layers (Furuse *et al.,* J. Cell Biol. *123*:1777-1788, 1993). Occludin functions to seal the tight junctions formed between cells in a cell layer. Because occludin is required to maintain the integrity of tight junctions, modulation of occludin synthesis would be desirable to enhance the paracellular permeability of cell layers and, thereby, the movement of solutes, including drugs, across these cell layers.

Thus far, the nucleotide sequences of full length cDNA encoding occludin from human (SEQ ID NO:1), mouse (SEQ ID NO:2), dog (SEQ ID NO:3), chicken (SEQ ID NO:4) (Furuse *et al.,* J. Cell Biol. 123:1777-1788, 1993) and kangaroo rat have been described (Ando-Akatsuka *et al.,* J. Cell Biol. *133*:43, 1996). The amino acid sequences of mammalian occludins from human, mouse and dog show approximately 90% homology, whereas the amino acid sequences from mammalian, chicken and rat kangaroo occludins show approximately 50% homology (Ando-Akatsuka *et al.,* J.Cell Biol. *133*:43, 1996).

The occludin protein comprises four transmembrane domains, a long carboxyl-terminal cytoplasmic domain, a short amino-terminal cytoplasmic domain, two extracellular loops and one intracellular turn. The nonpolar nature of the extracellular domains and the conservation of their sequences among human, mouse, dog, chicken and kangaroo rat occludin suggest the extracellular domains are important for occludin function.

In view of the location of occludin within tight junctions and the putative structure of occludin, two extracellular loops which face into the tight junction space, transient interference with either occludin synthesis or occludin function may result in a transient increase in paracellular permeability. Such a transient increase in paracellular permeability could enable an increase in drug absorption by paracellular transport across a cell layer with minimal toxic effects.

An antisense oligonucleotide is a sequence of single stranded DNA or RNA synthesized by chemical means *in vitro* which is "complementary" in sequence to a specific intracellular target DNA or RNA. Antisense oligonucleotides offer the potential to block the expression of specific genes or translation of specific mRNAs within cells. The hydrogen bonding of an antisense oligonucleotide to its complementary mRNA may prevent or block the translation of the mRNA to yield the coded protein by steric hindrance. Alternatively, the interaction between an antisense oligonucleotide and its complementary mRNA within mammalian cells may induce the destruction of that mRNA by RNase H or by other unknown or uncharacterized RNases.

Therefore, what is needed is a composition and method for transiently interfering with occludin mRNA translation so that occludin synthesis and, thereby, occludin function are transiently disrupted and paracellular permeability is transiently enhanced.

WO-A-9732982 and corresponding EP-A-0831148 relate to the human adhesion molecule occludin. These publications describe *inter alia* the antisense inhibition of human occludin synthesis in epithelial cells as a means of enhancing drug delivery through epithelial cell layers. However, no specific antisense sequence is disclosed.

Therefore, it is an object of the present invention to provide a composition and use thereof for transiently enhancing paracellular transport across a cell layer in an animal including a human.

It is another object of the present invention to provide a composition and use thereof for transiently disrupting the integrity of the tight junctions of an epithelial cell layer in an animal including humans.

It is another object of the present invention to provide a composition and use thereof for decreasing the amount of occludin in the tight junctions of a cell layer.

It is another object of the present invention to provide a composition and use thereof for disrupting the function of occludin in the tight junctions of a cell layer.

It is another object of the present invention to provide a composition and use thereof for transiently disrupting the integrity of the tight junctions of an endothelial cell layer in an animal including a human.

It is another object of the present invention to provide a composition and use thereof for enhancing drug movement across the gastrointestinal epithelium into the systemic circulation of an animal including a human.

It is another object of the present invention to provide a composition and use thereof for enhancing drug movement from the systemic circulation into the interstitial space in an animal including a human.

It is another object of the present invention to provide a composition and use thereof for enhancing drug movement across the pulmonary epithelium into the pulmonary circulation of an animal including a human.

It is another object of the present invention to provide a composition and use thereof for enhancing drug uptake across the blood brain barrier in an animal including a human.

It is another object of the present invention to provide a composition and use thereof for transiently interfering with the translation of occludin mRNA.

It is another object of the present invention to provide a composition and use thereof for transiently interfering with the transcription of the occludin gene.

It is another object of the present invention to provide an antisense oligonucleotide composition that is stable.

It is another object of the present invention to provide an antisense oligonucleotide composition that is able to enter the target cells.

It is another object of the present invention to provide an antisense oligonucleotide composition that is retained by the target cells.

It is another object of the present invention to provide an antisense oligonucleotide composition that is able to interact with its cellular target.

It is another object of the present invention to provide an antisense oligonucleotide composition that interacts specifically with its cellular target.

It is another object of the present invention to provide an antisense oligonucleotide composition that demonstrates low toxicity.

It is another object of the present invention to invention to provide an antisense oligonucleotide composition that can be synthesized easily.

These and other objects of the invention will become apparent from a review of the present specification.

Disclosure of Invention

The invention provides an antisense oligonucleotide consisting of the nucleic acid sequence of SEQ ID NO: 5 hybridizable with a region of occludin mRNA such that the translation of occludin is disrupted.

More particularly, the present invention provides a composition comprising an antisense oligonucleotide consisting of the nucleic acid sequence of SEQ ID NO: 5 hybridizable with a region of messenger RNA coding for the protein occludin (occludin mRNA) which, when hybridized to occludin mRNA, interferes with translation such that occludin function is disrupted and paracellular permeability is increased across an epithelial cell layer or an endothelial cell layer in an animal.

The present invention satisfies the above needs by providing a composition comprising an oligonucleotide consisting of the nucleic acid sequence of SEQ ID NO: 5 hybridizable with a region of occludin mRNA, or of occludin DNA, which interferes with the translation of the mRNA, or the transcription of the DNA, when hybridized to the mRNA, or the DNA, such that the synthesis of occludin is down-regulated. More specifically, the composition of the present invention provides an antisense oligonucleotide consisting of the nucleic acid sequence of SEQ ID NO: 5 which hybridizes with a region of occludin mRNA and, when hybridized to a region of occludin mRNA, interferes with its function such that occludin synthesis is disrupted. This antisense oligonucleotide, when administered in an effective concentration to an animal, including a human, transiently interferes with occludin translation so that occludin function in tight junctions is transiently disrupted, and so that the permeability of the cell layer to solutes, including drugs, is transiently increased.

Brief Description of Drawings

Fig. 1 shows the effect of occludin antisense oligonucleotide treatment on the flux of the hydrophilic marker [$^3$H]-mannitol across polarized Caco-2 monolayers after 90 minutes;
Fig. 2. shows the effect of occludin antisense oligonucleotide treatment on flux of the hydrophilic marker [$^3$H]-mannitol across polarized Caco-2 after 180 minutes; and
Fig. 3 shows the effect of occludin antisense oligonucleotide treatment on the apparent permeability of Caco-2 monolayers to the hydrophilic marker mannitol.

The antisense oligonucleotide sequence according to the invention can hybridize to a region of occludin mRNA or DNA. Such an antisense sequence is highly useful for interfering with normal occludin transcription or translation and, thereby, enhancing paracellular transport across cell layers in an animal, including a human.

The antisense oligonucleotide of this invention is complementary to a specific nucleic acid sequence of a region of occludin mRNA. More preferably, the antisense oligonucleotide is complementary to the translation initiation nucleic acid sequence of occludin mRNA. The resultant reduction in occludin protein disrupts tight junctions between the cells in cell layers so that the permeability of the cell layer to solutes, including drugs, is enhanced.

Generally, the oligonucleotide according to the invention will have a sequence exactly complementary to a nucleic acid sequence of a region of occludin mRNA. However, absolute complementarity is not required. Any nucleotide having sufficient complementarity to form a stable duplex with a region of occludin mRNA so that hybridization is possible and translation of the RNA is inhibited, for example by duplex formation or RNase H activation, is considered suitable. Stable duplex formation depends on the sequence and length of the oligonucleotide and the degree of complementarity between the antisense oligonucleotide and the target sequence. RNase H activation, on the other hand, may require only 5 contiguous matched base pairs (Monia *et al.,* J. Cell Biol. *268*:14514, 1993).

Any oligonucleotide which stably hybridizes to a region of occludin mRNA and which inhibits translation of the mRNA is considered effective. However, the region of occludin mRNA to which the antisense oligonucleotide hybridizes may affect the practice of this invention. That is, oligonucleotides complementary to specific regions of occludin mRNA including, but not limited to, the translation initiation nucleic acid sequence, the translation elongation nucleic acid sequence and the 3' untranslated region, are particularly effective. Oligonucleotides that inhibit splicing or inhibit 5' capping also may be effective for interfering with translation of occludin mRNA.

Also the oligonucleotide can be a stabilized oligonucleotide.

The stabilized oligonucleotide can be selected from an oligonucleotide stabilized by inclusion of o-methyl linkages, an oligonucleotide stabilized by inclusion of a nucleotide analog, an oligonucleotide stabilized by inclusion of a sugar analog and an olignucleotide stabilized by modification of the phosphodiester backbone.

The oligonucleotide for use in this invention may be unmodified or modified. Modifications are designed to increase resistance to nuclease attack *in vivo,* to increase specificity or to increase cellular uptake. Such modifications include, but are not limited to, phosphoramidite modifications (Gryaznon *et al.,* J. Am. Chem. Soc. *116*:3143, 1994), phosphorothioate modifications (La Planche *et al.,* Nucleic Acids Res. 14:9081, 1986), methyl phosphanate modifications and short chain alkyl or cycloalkyl modifications. Modifications also may include analogs having one or more modified base forms including purines and pyrimidines not found in nature, oligoribonucleotide analogs including, but not limited to, 2'-methylribonucleotides (Inoue *et al.,* Nucleic Acids Res. 15:6131, 1987), chimeric oligonucleotides (Inoue *et al.,* FEBS Letters 215:327, 1987), O-methyl linkages and other analogs known to those skilled in the art.

Therefore, to enhance the transfection of the occludin antisense oligonucleotide into the cell and into regions of the cell where the occludin mRNA is located, the occludin antisense oligonucleotide may be modified in a variety of ways known to those skilled in the art. However, any such modified oligonucleotide must be functionally interchangeable with the naturally occurring oligonucleotides. That is, it must hybridize effectively with occludin mRNA and interfere with its translation. Alternatively, such modified oligonucleotides may hybridize with the corresponding occludin DNA and interfere with its transcription.

The efficiency of cellular uptake of oligonucleotides may be increased by complexing the oligonucleotides or modified oligonucleotides with a cationic lipid or with a cationic liposome. Cationic lipids include, but are not limited to, lipofectamine, Lipofectin, DOTAP, Transfectam TransfectAce and GS-2888 (Lewis *et al.,* PNAS 93:3177, 1996). The oligonucleotide complexes with the cationic lipid or cationic liposome by ionic interactions and the fusogenic properties of the cationic lipids facilitate cellular uptake of the oligonucleotides. Alternatively, the oligonucleotides may be entrapped in the aqueous space of a liposome (MEV) such as, but not limited to, a cardiolipin:phosphatidylcholine:cholesterol (2: 10:7) liposome and then enter the cell by endocytic uptake of the liposome.

Other strategies may include, but are not limited to, conjugating the oligonucleotides with poly (L-lysine) (Leonettie *et al.,* Bioconjug. Chem. 1:149, 1990; Clarenc *et al.,* Anticancer Drug Design, 8:81), polyethylenime (Boussif *et al.,* PNAS, *92*:7279, 1995), other "interpolyelectrolyte complexes" (Kabanov *et al.,* Bioconjug. Chem. 6:7, 1995), fusogenic peptides (Plunket *et al.,* J. Biol. Chem. 269:12918, 1995; Bongartz *et al.,* Nucl. Acids Res. 22:4681, 1994) or peptide fragments of the homeodomain of the *Drosophila* antennaledia protein (Derossi *et al.,* J. Biol. Chem. *269*:10444, 1994), transferrin-polylysine conjugates, cholesterol (Ing *et al.,* Nucl. Acids Res. 21:2789, 1993), polyaminilipids including, but not limited to, spermidine-cholesterol or spermine-cholesterol, acridine, peptides and fatty acids. Strategies also include targeting oligonucleotides to cell-surface receptors such as folate (Wang *et al.,* PNAS 92:3318, 1995), asialoglycoprotein receptors (Wu *et al.,* J. Biol. Chem. 267:12436, 1992) and transferrin (Citro *et al.,* PNAS 89:7031, 1992) or to other receptors such as the vitamin $B_{12}$ receptor or the intrinsic factor that associates with the vitamin $B_{12}$ receptor. Targeting of oligonucleotides to specific cells via specific sugar-binding receptors or membrane lectins found on the surface of cells also is contemplated. For example, heme-coated cationic liposomes (Innovir Laboratories, New York) are taken up by liver Kuppfer cells, a small organic structure containing a hydrophobic moiety (INNOPHOR™, Innovir Laboratories, New York) is taken up by all liver cells and poly-lysine-adialoorosomucoid protein conjugates bind to liver-specific AsOR receptors. Further, protein A-bearing neutral liposomes may be used to selectively target cells by incubating them with specific monoclonal antibodies (Mabs).

The occludin antisense oligonucleotide according to the invention, either alone or after modification or conjugation, may be coupled to the surface of nanoparticles or can be entrapped within nanoparticles or microparticles to enhance the uptake thereof. Examples of nanoparticle carriers include, but are not limited to, cyanoacrylate nanoparticle systems, polylactide systems, polyglycolide systems or polylactide co-glycolide systems, etc.

Once within a cell, the effectiveness of the oligonucleotides can be enhanced by adjuvants that increase endosomal to cytosolic transfer including, but not limited, to fusogenic peptides, 5th generation Starburst dendrimers (Haensler *et al.,* Bioconjug. Chem. 4:372, 1994,), a pH responsive polymer poly(-ethylacrylic acid) (Tirrell *et al.,* Annal. New York Acad. Sci. *446:237,* 1985), cationic liposomes and a newly synthesised pH sensitive surfactant N-dodecyl 2-imidazole-propionate or DIP (Hughes *et al.,* Pharmac. Res. 13:404, 1996).

The occludin antisense oligonucleotide of this invention can be administered in a therapeutically effective amount to an animal, including a human, either alone or in combination with a pharmaceutically acceptable carrier. The term "therapeutically effective" means that the amount of occludin antisense oligonucleotide is of sufficient quantity to increase paracellular transport to some beneficial degree. The term "in combination" means that the occludin antisense oligonucleotide and the pharmaceutically acceptable carrier can be administered simultaneously, separately, at different frequencies or by different routes. The term "pharmaceutical combination" includes mixed associations of the occludin antisense oligonucleotide and pharmaceutically acceptable carriers and also non-mixed associations such as those found in kits or pharmaceutical packs.

The antisense oligonucleotide, alone or in combination with carriers, can be administered in a single dose or in multiple doses over a period of time. Administration can be oral, parenteral, mucosal, topical, transdermal, by implant, by minipump, by biodegradable polymer matrices, or by any other acceptable route known to those skilled in the art. Administration can be in combination with any biocompatible adjuvant, additive or carrier including, but not limited to, aqueous vehicles and nonaqueous vehicles. Oral administration includes, but is not limited to, tablets, suspensions, solutions, emulsions, capsules, powders, syrups and water compositions. For oral administration, antisense oligonucleotide delivery forms may require some form of enteric coating to prevent their digestion or degradation prior to their absorption in the gastrointestinal tract. In addition, suitable delivery formulation and enteric coatings are contemplated which enable targeted release and sustained delivery in the gastrointestinal tract. Parenteral administration includes, but is not limited to, injection and infusion. Mucosal administration includes, but is not limited to, solutions and sprays. Various additives which enhance the stability, sterility and isotonicity of the composition including, but not limited to, anti microbial preservatives, antioxidants, chelating agents, gelatin and buffers may be added as long as they are compatible with the occludin antisense oligonucleotide.

Dosage ranges for the administration of the occludin antisense oligonucleotide according to the invention are those sufficient to produce the desired effect of enhancing paracellular transport. The dosage should not be so large as to cause adverse side effects such as anaphylactic or unwanted cross-reactions or an immune response. Generally, the dosage will vary with the age, condition and sex of the animal, with the physical and chemical properties of the therapeutic agent to be absorbed by paracellular transport, with the cell layer across which the therapeutic agent must be transported, for example the intestinal tract, the lung epithelium, or the blood-brain barrier and with the route of administration. Preferably, dosage of occludin antisense oligonucleotides is above approximately 100 μM, more preferably above approximately 250 μM, and most preferably above approximately 500 μM. However, one skilled in the art can determine the particular therapeutically effective dose of the occludin antisense oligonucleotide to be used depending upon the circumstances in each case without undue experimentation.

The effect of the occludin antisense oligonucleotide for use in this invention is transient. That is, it interferes with translation of occludin mRNA and enhances paracellular permeability for a limited time. Generally, the amount of time will vary with the physical and chemical properties of the therapeutic agent to be absorbed by paracellular transport. It will also vary with the severity of the disorder to be treated. Preferably, the occludin antisense oligonucleotide will interfere with translation of occludin mRNA so that paracellular permeability is increased from approximately thirty minutes to approximately fourteen days, and more preferably from about one hour to about seven days. However, one skilled in the art can determine the period of time necessary for paracellular absorption of an effective amount of a therapeutic agent without undue experimentation.

The oligonucleotide for use in this invention consists of the sequence SEQ ID NO: 5 of 17 subunits. A "subunit" means a nucleotide base and sugar combination (or a nucleotide analog and/or sugar analog combination) suitably bound to adjacent subunits through phosphodiester or other bonds.

The oligonucleotide of this invention is hybridizable with a region within occludin mRNA and interferes with translation of the mRNA. The functions of mRNA which may be interfered with include, but are not limited to, translocation of the RNA to the site of protein translation, actual translation of the protein from the mRNA decreased stability of the mRNA due to activation of RNases such as RNaseH and possible catalytic activity of the mRNA. Interference with mRNA translation disrupts occludin synthesis and enhances paracellular transport across cell layers in an animal.

Generally, the oligonucleotide of SEQ ID NO: 5 which hybridizes with occludin mRNA or with the parent occludin gene can be used to disrupt occludin synthesis and, thereby, enhance paracellular transport. Triple-helix forming oligonucleotides which hybridize with or interact with sequences within the occludin gene or the promoter which regulates or controls the expression of the occludin gene will also have the desired effect of reducing the amount of occludin mRNA and disrupting occludin synthesis. Preferred are oligonucleotides which hybridize with occludin mRNA: More preferred is an oligonucleotide which hybridizes with the translation initiation region of occludin mRNA or with those regions of the occludin mRNA which will maximally reduce occludin synthesis, for example, within the coding sequence, the 3' untranslated region, or the 5' untranslated region of the mRNA.

The oligonucleotide according to the invention may be useful in diagnostics, therapeutics and as research reagents. For therapeutic use, the oligonucleotide is administered to an animal, including a human, either prior to or at the same time as a therapeutic agent to enhance uptake of the therapeutic agent through paracellular pathways.

The invention also provides use of the antisense oligonucleotide of SEQ ID NO: 5 in the manufacture of a medicament for use in a method for enhancing the delivery of a therapeutic agent across a cell layer by paracellular transport, wherein the medicament is administered to an animal in an amount effective to interfere with the translation of occludin such that paracellular permeability of the therapeutic agent across the cell layer is transiently increased.

Any therapeutic agent capable of being administered via epithelial or endothelial cell barriers is encompassed by this invention. Preferably such therapeutic agents include, but are not limited to, autonomic drugs, cardiovascular drugs, pulmonary drugs, gastrointestinal drugs, renal drugs, central nervous system drugs, chemotherapeutic drugs, and dermatologic drugs. More preferably, such therapeutic agents include, but are not limited to, therapeutic agents designed to be absorbed through paracellular pathways.

The drug can be selected from a peptide, a protein; a gene delivery vector and a drug with low permeability across endothelial or epithelial cell layer.

Therapeutic agents can be administered before, during or after administration of the antisense oligonucleotide. Preferably, therapeutic agents are administered simultaneously with or within 12, 24 or 48 hours following administration of the antisense oligonucleotide.

An alternative approach to decreasing occludin synthesis is to down-regulate the gene coding for occludin mRNA. To do this, a gene which codes for an RNA sequence complementary to the endogenous naturally occurring mRNA coding for occludin (cRNA) is introduced into a cell such as, but not limited to, a jejunal epithelial cell, an ileal epithelial cell, or a capillary endothelial cell. The introduced gene will code for the cRNA sequence which is complementary to the mRNA coding for occludin. Base-pairing of the cRNA with the mRNA within the cell will down-regulate the expression of occludin protein through this cRNA:mRNA interaction.

The gene coding for the cRNA can be introduced into mammalian cells in a viral vector delivery system such as, but not limited to, an adenovirus, an adeno-associated viral vector system, a retrovirus, a herpes simplex virus, or any other gene delivery system known to those skilled in the art.

The gene coding for the cRNA also can be introduced into cells as naked DNA or as naked plasmid molecules containing the gene coding for the cRNA using non-viral means such as, but not limited to, liposomes, lipid-based transmembrane carriers, cytofectins and by encapsulation into polymer systems such as, but not limited to, dendrimer polymer systems, PLGA polymer systems, polycyanoacrylate polymer systems and other polymer systems.

In addition, the gene coding for the cRNA can be associated with chimeric fusion proteins whereby one region of the fusion protein is a DNA binding peptide or protein such as, but not limited to, poly-L-lysine, which binds to the naked DNA or plasmid molecules containing the gene of interest and a second region of the chimeric fusion protein bind to and targets chosen receptor sites in, for example, the epithelial cells of the gastrointestinal tract such as, but not limited to, the vitamin B 12 transporter, the glucose transporter, the HPT-1 receptor, the PEPT1 transporter, the D2H protein, the human sucrase-isomaltase complex, the folate receptor or any other receptor expressed on the apical membrane of epithelial cells. Likewise, the second region of the chimeric fusion protein can bind to and target chosen receptor sites expressed on the apical membrane of endothelial cells.

Furthermore, the chimeric fusion protein may contain a third region termed a Nuclear Localization Signal or Nuclear Localization Sequence (NLS) which will traffic the complex of the chimeric fusion protein associated with the gene/DNA/plasmid molecule of interest to the nucleus of the cell once this complex has traversed the plasma membrane and entered the cytoplasm of the cell.

Synthetic peptides of both linear and cyclic conformation corresponding to extracellular loop domains of occludin also may be used to interfere with occludin function in the tight junctions of cell layers and, thereby, to increase paracellular transport of therapeutic agents across a cell layer.

The following examples will serve to further illustrate the present invention without, at the same time, constituting any limitation on the present invention. On the contrary, it is to be clearly understood that resort may be had to various other embodiments, modifications and equivalents thereof which, after reading the description herein, may suggest themselves to those skilled in the art without departing from the spirit of the present invention and/or the scope of appended claims.

<u>Modes for Carrying Out the Invention</u>

EXAMPLE 1

*Culture of Caco-2 Cells:*

Human intestinal Caco-2 cells were grown using standard techniques well known to those skilled in the art. The cells were seeded onto polycarbonate filters (Costar Snapwells; diameter = 12 mm; area =1.13 $cm^2$; pore size = 0.4m) at a density of 0.5 x $10^6$ $cm^2$. For antisense oligonucleotide studies, 18-25 day old cell monolayers were used which have transepithelial electrical resistance (TER) measurements in the range 200-400 $cm^2$.

EXAMPLE 2

*Antisense oligonucleotide treatment:*

All incubations were at 37°C in 5% $CO_2$ atmosphere. All solutions were warmed to 37°C before use. The following procedure was carried out once a day over three days. After reading the TER, the cells were washed once and incubated in a reduced serum medium (RSM; Opti-MEM 1 with GlutaMax 1; Gibco-BRL) for 30 minutes. Meanwhile, the lipofectamine-oligonucleotide complex was allowed to form at room temperature. Lipofectamine (20μl/snapwell; Gibco BRL CN. 18324-012) was mixed with occludin antisense DNA oligonucleotide, with occludin scrambled oligonucleotide or with water control in RSM. Antisense oligonucleotides (phosphorothioate oligonucleotides) synthesized by Genosys (Cambridge, UK) were designed to hybridize with the translation initiation region of human occludin mRNA, as follows:

Occludin antisense oligonucleotide used is:
5' AAG AGG CCT GGA TGA CA 3' (SEQ ID NO: 5)
Occludin scrambled oligonucleotide used is:
5' GCA AGT CAG GAC GTA GA 3' (SEQ ID NO:6)
The lipofectin:oligonucleotide complex was added to the apical surface of the snapwells and the cells were incubated for 2 hours. The apical and basolateral media were then replaced with normal medium (except on day 4). After the 2 hour incubation on day 3, transepithelial flux of $^3$H-mannitol was measured.

EXAMPLE 3

*$^3$H-mannitol flux:*

Caco-2 cells were washed once in buffered Hank's balanced salt solution (bHBSS; Gibco CN.14065-031; supplemented with 0.011M glucose, 15 mM HEPES acid [3.575g/l; Sigma CN.H3375], 10 mM HEPES base [2.603g/l; Sigma CN. H1016]) and were incubated 30 minutes. The apical reservoir bHBSS was replaced with 1 ml buffered HBSS containing 1 μCi/ml of the hydrophilic marker $^3$H-mannitol (New England Biolabs). A 10 μl sample of the stock solution was retained for later measurement and 10 μl apical samples were taken at the end of the experiment. One ml samples from the basolateral reservoirs were taken at appropriate time points (90 minutes or 120 minutes post addition of $^3$H-mannitol). After each basolateral sampling, the snapwells were placed in fresh 6-well plates and 2 ml of prewarmed bHBSS was added to the basolateral reservoir. The samples were added to 4 ml scintillation fluid and radioactivity for each vial counted on a scintillation counter.

EXAMPLE 4

*Treatment of cultured human epithelial cells with occludin antisense oligonucleotide.*

Fully differentiated Caco-2 monolayers were grown as in Example 1 and were subjected to antisense treatment as in Example 2.

Table 1 shows the number of passages and the age of the cells used in the studies detailed in Experiment 1 in Example 5 and Experiment 2 in Example 6.

Table 1

| | Passage | Age of Cells |
|---|---|---|
| Experiment 1 | 32 | 16 |
| Experiment 2 | 42 | 28 |

EXAMPLE 5

*Effect of occludin antisense treatment on mannitol flux across Caco-2 cells*

Fully differentiated Caco-2 monolayers were grown as in Example 1. The cells were treated over 3 days with lipofectamine alone, lipofectamine and occludin antisense oligonucleotide (Sequence ID No.5) or lipofectamine and occludin scrambled oligonucleotide, (Sequence ID No.6) at 5 $\mu$M on day 1, 50 $\mu$M on day 2 and 500 $\mu$M on day 3 as in Example 2. On day 4, [$^3$H]-mannitol was applied to the apical domain and flux was determined by measuring the radioactivity (cpm) transferred to the basolateral reservoir over 90 minutes as in Example 3.

Table 2 and Figure 1 show the effect of lipofectamine alone of lipofectamine + occludin antisense oligonucleotide (SEQ ID NO:5) and of lipofectamine + occludin scrambled oligonucleotide (SEQ ID NO: 6) on [$^3$H]-mannitol flux in polarized Caco-2 cells measured after 90 minutes as in Example 3.

Table 2

[$^3$H]-mannitol flux in polarized Caco-2 cells.

| *Treatment* | *Snapwells* | *TER* | *(cpm/ml)* | *Mean* | *S.D* |
|---|---|---|---|---|---|
| Lipofectamine | 1 | 1050 | 12955 | | |
| | | | 13340 | | |
| | 2 | 1220 | 12290 | | |
| | | | 12300 | | |
| | | | | 12721 | 3385.7 |
| Lipofectamine + Occludin | 3 | 1260 | 19463 | | |
| | | | 26644 | | |
| Antisense | 4 | 1410 | 17550 | | |
| | | | 19310 | | |
| | | | | 20742 | 6402.5 |
| Lipofectamine + Scrambled | 5 | 1280 | 12898 | | |
| | | | 13015 | | |
| Antisense | 6 | 1700 | 14075 | | |
| | | | 12094 | | |
| | | | | 13020 | 2527.5 |
| Apical Reservoir (pooled) | | | 4845771 | | |
| | | | 4943129 | | |

Figure 1 shows the effect of lipofectamine alone of lipofectamine + occludin antisense (SEQ ID NO:5) and of lipofectamine + occludin scrambled (SEQ ID NO:6) on [$^3$H]-mannitol flux in polarized Caco-2 cells measured after 90 minutes as in Example 3.

The data in Table 2 and in Figure 1 demonstrate that treatment of Caco-2 cells with occludin antisense oligonucleotide lead to a 1.6 fold increase in flux relative to no oligonucleotide and to a 1.6 fold increase in flux relative to occludin scrambled oligonucleotide.

EXAMPLE 6

*Effect of occludin antisense treatment on mannitol flux across Caco-2 cells*

Lipofectamine (20 μl/snapwell; Gibco BRL CN. 18324-012) was mixed with 500 μM of occludin antisense (SEQ ID NO:5) or of water control in RSM and was added to the cells as in Example 2.

The synthetic phosphorothioate oligonucleotides were complementary to the translation initiation region of human occludin mRNA (SEQ ID NO:5) and were designed to inhibit translation of the occludin protein. A negative control oligonucleotide, occludin scrambled (SEQ ID NO:6) was also used.

Table 3 shows the effect of lipofectamine (Lip), of lipofectamine + occludin scrambled (Occ Neg), of lipofectamine + occludin antisense (Occ Pos) and of water (No Treatment) on [$^3$H]-mannitol flux in polarized Caco-2 cells measured after 90 minutes as in Example 3.

Table 3

$^3$H mannitol flux in polarized Caco-2 cells

| | Lip | Occ Neg | Occ Pos | No Treatment |
|---|---|---|---|---|
| 1 | 1743 | 1789 | 2058 | 1629 |
| 2 | 1964 | 1948 | 4245 | 1657 |
| 3 | 2547 | 1815 | 4266 | 1822 |
| 4 | 2963 | 1666 | 4918 | 2654 |
| 5 | 1861 | 1738 | 4662 | 2065 |
| 6 | 3743 | 2498 | 5135 | 1122 |
| av | 2470.167 | 1909 | 4214 | 1824.833 |
| sd | 777.8114 | 303.3045 | 1113.244 | 511.0184 |

Figure 2 shows the effect of lipofectamine, of lipofectamine + occludin scrambled (SEQ ID NO:6), of lipofectamine + occludin antisense (SEQ ID NO:5) and of water on the flux of the [$^3$H]-mannitol in Caco-2 monolayers grown on snapwells as in Example 2. In this experiment, antisense treatment led to a mean 2.2-fold increase in [$^3$H]-mannitol flux relative to control scrambled sequence antisense DNA. The cells were treated over 3 days with lipofectamine alone, lipofectamine + occludin scrambled, lipofectamine + occludin antisense and water. On day 4, [$^3$H]-mannitol was applied to the apical domain and the fluxes were determined by measuring the radioactivity (dpm) transferred to the basolateral reservoir over 120 minutes as in Example 3.

Figure 3 shows the apparent permeability values (Papp) of the polarized Caco-2 cells to [$^3$H]-mannitol. The cells were treated with lipofectamine, lipofectamine + 500 μM occludin scrambled (SEQ ID NO:6), lipofectamine + 500 μM occludin antisense (SEQ ID NO:5) or water. Apparent permeability values (Papp) were calculated according to the equation

$$Papp = (dQ/dt)(1/C.A)$$

where
dQ/dt = flux rate (mmol/s)
C = concentration on donor side at t = 0 mmol/cm$^3$)
A = area of monolayer (cm$^2$).

The apparent permeability of the monolayers was 415.78 cm s$^{-1}$ for cells treated with the specific occludin antisense DNA compared to 188.35 cm s$^{-1}$ for cells treated with a scrambled control oligonucleotide. This difference is statistically significant (p=0.0045; 2-tailed unpaired t test). These data demonstrate that an antisense oligonucleotide targeted to human occludin mRNA mediates increased paracellular permeability from apical to basolateral compartments in cultured human intestinal epithelial cells.

**Claims**

1. An antisense oligonucleotide consisting of the nucleic acid sequence of SEQ ID NO:5.

2. An oligonucleotide according claim 1, wherein the oligonucleotide is a stabilized oligonucleotide.

3. An oligonucleotide according to claim 2, wherein the stabilized oligonucleotide is selected from an oligonucleotide stabilized by inclusion of o-methyl linkages, an oligonucleotide stabilized by inclusion of a nucleotide analog, an oligonucleotide stabilized by inclusion of a sugar analog and an olignucleotide stabilized by modification of the phosphodiester backbone.

4. A pharmaceutical composition comprising: a pharmaceutically acceptable carrier; and an antisense oligonucleotide according to any one of claims 1-3.

5. A product containing an antisense oligonucleotide according to any one of claims 1-3 and a therapeutic agent as a combined preparation for simultaneous, separate or sequential use in a method for enhancing the delivery of said therapeutic agent across a cell layer by paracellular transport, said oligonucleotide is to be administered to an animal in an amount effective to interfere with the translation of occludin such that paracellular permeability of the therapeutic agent across the cell layer is transiently increased.

6. A product according to claim 5, wherein the cell layer is an epithelial cell layer.

7. A product according to claim 5, wherein the cell layer is an endothelial cell layer.

8. A product according to any one of claims 5-7, wherein the oligonucleotide is complementary to the occludin mRNA translation initiation region.

9. A product according to any one of claims 5-8, wherein the therapeutic agent is a drug.

10. A product according to claim 9, wherein the drug is selected from those drugs to be administered across an epithelial cell layer.

11. A product according to claim 9, wherein the drug is selected from those drugs to be administered across an endothelial cell layer.

12. A product according to any one of claims 9-11, wherein the drug is selected from a peptide, a protein, a gene delivery vector and a drug with low permeability across an endothelial or epithelial cell layer.

13. A product according to any one of claims 9-12, wherein the drug is to be administered simultaneously with the antisense oligonucleotide.

14. A product according to any one of claims 9-12, wherein the drug is to be administered after the antisense oligonucleotide.

## Patentansprüche

1. Antisens-Oligonukleotid, bestehend aus der Nukleinsäuresequenz von SEQ ID NR:5.

2. Oligonukleotid nach Anspruch 1, wobei das Oligonukleotid ein stabilisiertes Oligonukleotid ist.

3. Oligonukleotid nach Anspruch 2, wobei das stabilisierte Oligonukleotid ausgewählt ist aus einem durch Einschluss von o-Methylverknüpfungen stabilisierten Oligonukleotid, einem durch Einschluss eines Nukleotidanalogons stabilisierten Oligonukleotid, einem durch Einschluss eines Zuckeranalogons stabilisierten Oligonukleotid und einem durch Modifikation des Phosphodiestergerüsts stabilisierten Oligonukleotid.

4. Pharmazeutische Zusammensetzung, umfassend: einen pharmazeutisch verträglichen Träger und ein Antisens-Oligonukleotid nach einem der Ansprüche 1-3.

5. Produkt, enthaltend ein Antisens-Oligonukleotid nach einem der Ansprüche 1-3 und ein Therapeutikum als kombiniertes Präparat zur gleichzeitigen, getrennten oder aufeinander folgenden Verwendung in einem Verfahren zum Verbessern der Abgabe des Therapeutikums durch eine Zellschicht hindurch durch parazellulären Transport, wobei das Oligonukleotid in einer zum Beeinflussen der Translation von Occludin wirksamen Menge einem Tier zu verabreichen ist, so dass die parazelluläre Durchdringungsfähigkeit des Therapeutikums durch die Zellschicht hindurch vorübergehend erhöht wird.

6. Produkt nach Anspruch 5, wobei die Zellschicht eine epitheliale Zellschicht ist.

7. Produkt nach Anspruch 5, wobei die Zellschicht eine endotheliale Zellschicht ist.

8. Produkt nach einem der Ansprüche 5-7, wobei das Oligonukleotid zu der mRNA-Translationsinitiierungsregion von Occludin komplementär ist.

9. Produkt nach einem der Ansprüche 5-8, wobei das Therapeutikum ein Arzneimittel ist.

10. Produkt nach Anspruch 9, wobei das Arzneimittel ausgewählt ist aus denjenigen Arzneimitteln, die durch eine epitheliale Zellschicht hindurch zu verabreichen sind.

11. Produkt nach Anspruch 9, wobei das Arzneimittel ausgewählt ist aus denjenigen Arzneimitteln, die durch eine endotheliale Zellschicht hindurch zu verabreichen sind.

12. Produkt nach einem der Ansprüche 9-11, wobei das Arzneimittel ausgewählt ist aus einem Peptid, einem Protein, einem Genabgabevektor und einem Arzneimittel mit geringer Durchdringungsfähigkeit durch eine endotheliale oder epitheliale Zellschicht hindurch.

13. Produkt nach einem der Ansprüche 9-12, wobei das Arzneimittel gleichzeitig mit dem Antisens-Oligonukleotid zu verabreichen ist.

14. Produkt nach einem der Ansprüche 9-12, wobei das Arzneimittel nach dem Antisens-Oligonukleotid zu verabreichen ist.

## Revendications

1. Oligonucléotide anti-sens constitué par la séquence d'acide nucléique SEQ ID N° : 5.

2. Oligonucléotide selon la revendication 1, dans lequel l'oligonucléotide est un oligonucléotide stabilisé.

3. Oligonucléotide selon la revendication 2, dans lequel l'oligonucléotide stabilisé est choisi parmi un oligonucléotide stabilisé par inclusion de groupes de liaison o-méthyle, un oligonucléotide stabilisé par inclusion d'un nucléotide analogue, un oligonucléotide stabilisé par inclusion d'un sucre analogue et un oligonucléotide stabilisé par modification du squelette phosphodiester.

4. Composition pharmaceutique comprenant : un véhicule acceptable sur le plan pharmaceutique ; et un oligonucléotide anti-sens selon l'une quelconque des revendications 1 à 3.

5. Produit contenant un oligonucléotide anti-sens selon l'une quelconque des revendications 1 à 3 et un agent thérapeutique tel qu'une préparation combinée pour une utilisation simultanée, séparée ou séquentielle dans un procédé permettant de renforcer le transfert dudit agent thérapeutique à travers une couche cellulaire par transport paracellulaire, ledit oligonucléotide est destiné à être administré à un animal en quantité efficace pour interférer avec le déplacement de l'occludine de sorte que la perméabilité paracellulaire de l'agent thérapeutique à travers la couche cellulaire augmente de manière transitoire.

6. Produit selon la revendication 5, dans lequel la couche cellulaire est une couche de cellules épithéliales.

7. Produit selon la revendication 5, dans lequel la couche cellulaire est une couche de cellules endothéliales.

8. Produit selon l'une quelconque des revendications 5 à 7, dans lequel l'oligonucléotide est complémentaire à la région d'initiation de la traduction de l'ARNm de l'occludine.

9. Produit selon l'une quelconque des revendications 5 à 8, dans lequel l'agent thérapeutique est un médicament.

**10.** Produit selon la revendication 9, dans lequel le médicament est choisi parmi les médicaments destinés à être administrés à travers une couche de cellules épithéliales.

**11.** Produit selon la revendication 9, dans lequel le médicament est choisi parmi les médicaments destinés à être administrés à travers une couche de cellules endothéliales.

**12.** Produit selon l'une quelconque des revendications 9 à 11, dans lequel le médicament est choisi parmi un peptide, une protéine, un vecteur de transfert de gènes et un médicament de faible perméabilité à travers une couche de cellules endothéliales ou épithéliales.

**13.** Produit selon l'une quelconque des revendications 9 à 12, dans lequel le médicament est destiné à être administré simultanément avec l'oligonucléotide anti-sens.

**14.** Produit selon l'une quelconque des revendications 9 à 12, dans lequel le médicament est destiné à être administré après l'oligonucléotide anti-sens.

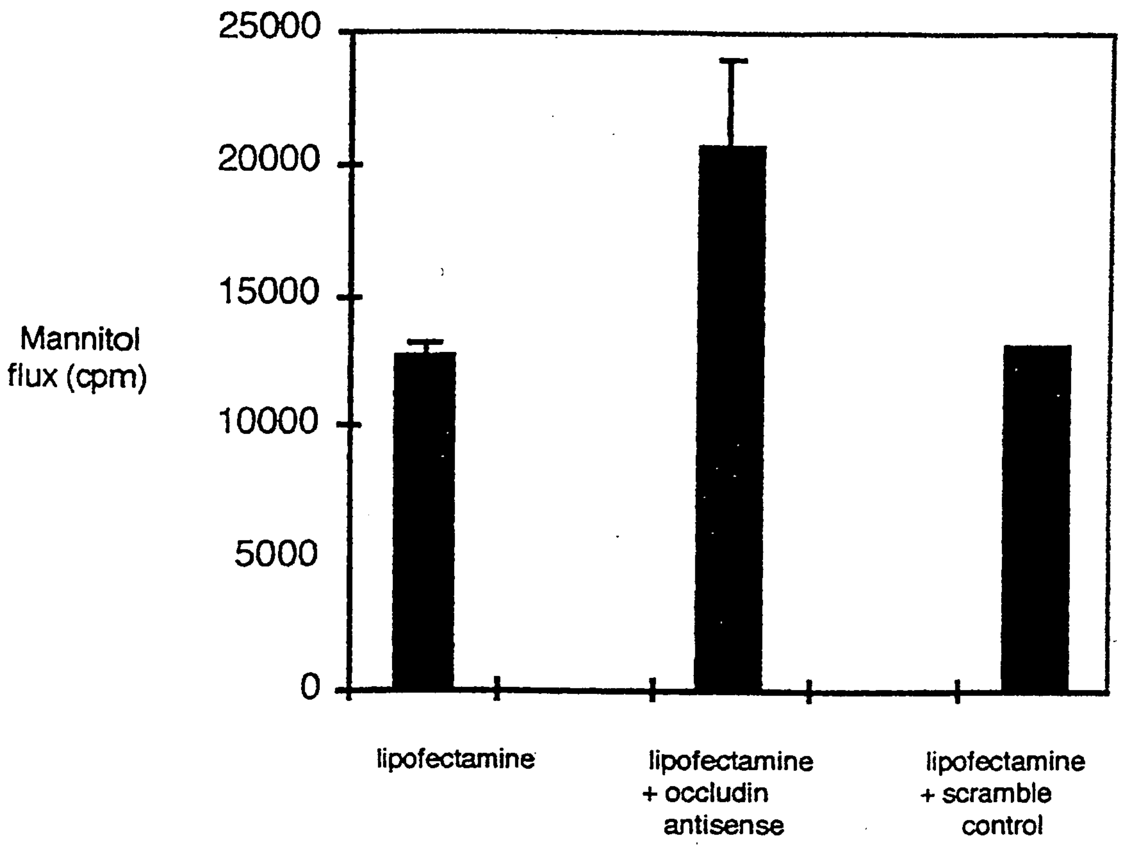
25000
20000
15000
10000
5000
0
Mannitol flux (cpm)
lipofectamine
lipofectamine + occludin antisense
lipofectamine + scramble control

FIG. 1

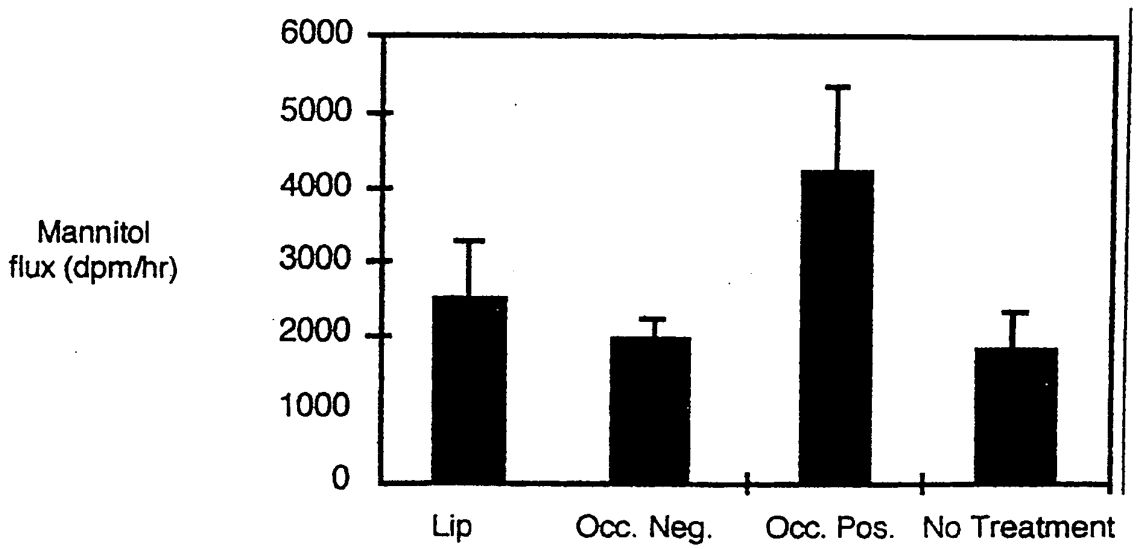
Mannitol
flux (dpm/hr)
6000
5000
4000
3000
2000
1000
0
Lip
Occ. Neg.
Occ. Pos.
No Treatment

FIG. 2

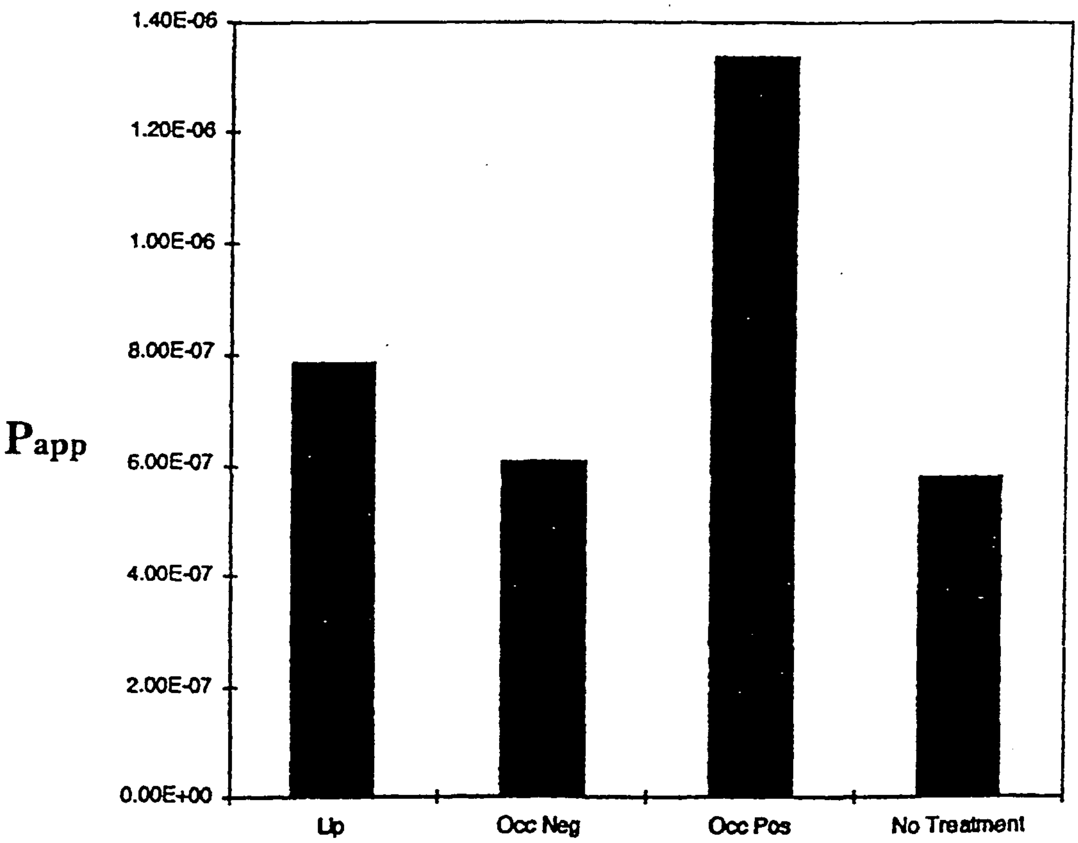
1.40E-06
1.20E-06
1.00E-06
8.00E-07
6.00E-07
4.00E-07
2.00E-07
0.00E+00
Papp
Up
Occ Neg
Occ Pos
No Treatment

FIG. 3

SEQUENCE LISTING

<110> ELAN CORPORATION, PLC

<120> Composition and method for enhancing paracellular transport across cell layers

<130> P98-198-PCT

<140>
<141>

<150> US 60/059,644
<151> 1997-09-24

<150> IE 970794
<151> 1997-11-10

<160> 6

<170> PatentIn Ver. 2.0

<210> 1
<211> 2377
<212> DNA
<213> Homo sapiens

<300>
<302> Interspecies Diversity of the Occludin Sequence: cDNA Cloning of Human, Mouse, Dog, and Rat-Kangaroo Homologues
<303> J. Cell Biol.
<304> 133
<305> 1
<306> 43-47
<307> April 1996
<308> U41984, U49221, U41985

<400> 1

ctcccgcgtc cacctctccc tccctgcttc ctctggcgga ggcggcagga accgagagag 60

gtccagagcg ccgaggagcc ggtctaggac gcagcagatt ggtttatctt ggaagctaaa 120

gggcattgct catcctgaag atcagctgac cattgacaat cagccatgtc atccaggcct 180

cttgaaagtc cacctcctta caggcctgat gaattcaaac cgaatcatta tgcaccaagc 240

aatgacatat atggtggaga gatgcatgtt cgaccaatgc tctctcagcc agcctactct 300

ttttacccag aagatgaaat tcttcacttc tacaaatgga cctctcctcc aggagtgatt 360

cggatcctgt ctatgctcat tattgtgatg tgcattgcca tctttgcctg tgtggcctcc 420

acgcttgcct gggacagagg ctatggaact tcccttttag gaggtagtgt aggctaccct 480

tatggaggaa gtggctttgg tagctacgga agtggctatg gctatggcta tggttatggc 540

tatggctacg gaggctatac agacccaaga gcagcaaagg gcttcatgtt ggccatggct 600

gccttttgtt tcattgccgc gttggtgatc tttgttacca gtgttataag atctgaaatg 660

tccagaacaa gaagatacta cttaagtgtg ataatagtga gtgctatcct gggcatcatg 720

gtgtttattg ccacaattgt ctatataatg ggagtgaacc caactgctca gtcttctgga 780

tctctatatg gttcacaaat atatgccctc tgcaaccaat tttatacacc tgcagctact 840

ggactctacg tggatcagta tttgtatcac tactgtgttg tggatcccca ggaggccatt 900

gccattgtac tggggttcat gattattgtg gcttttgctt taataatttt ctttgctgtg 960

aaaactcgaa gaaagatgga caggtatgac aagtccaata ttttgtggga caaggaacac 1020

atttatgatg agcagccccc caatgtcgag gagtgggtta aaaatgtgtc tgcaggcaca 1080

caggacgtgc cttcaccccc atctgactat gtggaaagag ttgacagtcc catggcatac 1140

tcttccaatg gcaaagtgaa tgacaagcgg ttttatccag agtcttccta taaatccacg 1200

ccggttcctg aagtggttca ggagcttcca ttaacttcgc ctgtggatga cttcaggcag 1260

cctcgttaca gcagcggtgg taactttgag acaccttcaa aaagagcacc tgcaaaggga 1320

agagcaggaa ggtcaaagag aacagagcaa gatcactatg agacagacta cacaactggc 1380

ggcgagtcct gtgatgagct ggaggaggac tggatcaggg aatatccacc tatcacttca 1440

gatcaacaaa gacaactgta caagaggaat tttgacactg gcctacagga atacaagagc 1500

ttacaatcag aacttgatga gatcaataaa gaactctccc gtttggataa agaattggat 1560

gactatagag aagaaagtga agagtacatg gctgctgctg atgaatacaa tagactgaag 1620

caagtgaagg gatctgcaga ttacaaaagt aagaagaatc attgcaagca gttaaagagc 1680

aaattgtcac acatcaagaa gatggttgga gactatgata gacagaaaac atagaaggct 1740

gatgccaagt tgtttgagaa attaagtatc tgacatctct gcaatcttct cagaaggcaa 1800

atgactttgg accataaccc cggaagccaa acctctgtga gcatcacaaa gttttggttg 1860

ctttaacatc atcagtattg aagcatttta taaatcgctt ttgataatca actgggctga 1920

acactccaat taaggatttt atgctttaaa cattggttct tgtattaaga atgaaatact 1980

gtttgaggtt tttaagcctt aaaggaaggt tctggtgtga actaaacttt cacaccccag 2040

acgatgtctt catacctaca tgtatttgtt tgcataggtg atctcattta atcctctcaa 2100

ccacctttca gataactgtt atttataatc actttttcc acataaggaa actgggttcc 2160

tgcaatgaag tctctgaagt gaaactgctt gtttcctagc acacactttt ggttaagtct 2220

gttttatgac ttcattaata ataaattccc tggcctttca tattttagct actatatatg 2280

tgatgatcta ccagcctccc tattttttt ctgttatata aatggttaaa agaggttttt 2340

cttaaataat aaagatcatg taaaagtaaa aaaaaaa 2377

<210> 2
<211> 1961
<212> DNA
<213> Canis familiaris

<300>
<302> Interspecies Diversity of the Occludin Sequence: cDNA Cloning of Human, Mouse, Dog, and Rat-Kangaroo Homologues
<303> J. Cell Biol.
<304> 133
<305> 1
<306> 43-47
<307> April 1996
<308> U41984, U49221, U41985

<400> 2

caggttggct tattttgggg agctctggga tcctgctcgt cctgaagatc gggtgatcat 60

tgacatcagc catgtcatcg aggccttttg agagtccacc tccgtataga cctgatgaat 120

tcaaacccaa tcattatgca ccgagcaatg atgtgtacgg tggggacatg cacgtccgac 180

ccatgctctc tcagccggcg tattctttct acccagaaga tgaaattctt cacttctaca 240

aatggacctc tcctccagga gtaattcgga ttctgtccat gcttgtcatt gtgatgtgca 300

tcgccatatt tggctgtgtc gcgtccacgc tcgcctggga tagaggctat ggaactggct 360

taatgggtgg tagcataggc tacccttacg gaagtggctt cgggagctac gggactggct 420

acggctacgg gtttggctac ggctacggct acggcggcta cacggatccc agagcagcaa 480

agggcttcct cctggccatg gtggcctttt gttttatcgc tgcattggtg atatttgtta 540

ccagcgttat aaggtctgac atatccagaa ccagaaggta ctacttgact gtaataatac 600

tgagtgcctt cctgggcgtc atgatgttca ttgctacaat tgtctatata atgggagtca 660

atccaactgc ccaggcttct gggtctttat acagttcaca gatatatgcc atgtgcaacc 720

agttctatgc atctacagct accggactct acatggatca gtatttgtat cactactgtg 780

tggtggatcc ccaagaggca attgccattg tcctgggatt catggtgatt gtggcttttg 840

ctttaataat tttctttgct gtgaaaactc gaagaaagat ggaccggtat gacaagtcga 900

atatattgtg ggacaaggaa catatttatg atgaacaacc ccccaatgtt gaagagtggg 960

ttaaaaacgt ttctgcaggc acacaagaca tgcctcctcc cccttctgac tatgtggaga 1020

gagtggacag tcccatggcg tactcttcca atggtaaagt gaatgacaag cggttgtatc 1080

cagagtcttc ctataaatca acaccggtcc ccgaagtggt gcaggagctg cccgccacct 1140

cccctgcgga tgacttcagg cagcctcgct acagcagcag cgggcacttg gagccacctt 1200

cgaagagggc cccctcgaaa ggaagaacgg gaaggcccaa gaggctggag caggaccact 1260

atgagacaga ctacacgacg ggcggcgagt cgtgtgacga gctggaggag gactggatca 1320

gggaatatcc acctatcact tcagatcaac aaagacaact ctacaagaga aattttgaca 1380

ctggcctgca ggaatacaag agcttacaag cagaacttga tgagatcaat aaagaactct 1440

ctcgcctgga taaagaattg gatgactata gagaagaaag tgaagagtac atggctgctg 1500

ctgatgagta caatagactg aagcaagtta agggatctcc agattacaaa aataagagga 1560

attattgcaa gcagttgaag agcaaattgt cccacatcaa gaagatggtt ggagactatg 1620

atagacagaa aacatagaag gcagatgcca cacagtttga gagattgtga agtatttgac 1680

atatctgcaa cgttgtcaga aggcagaatg actttggatt tcgaacccag gaggccagat 1740

ctttgtgatc attacaaagt tttggtagct ttaatatcat cagtattgaa gcattttaca 1800

catagctttt gataatcaac tgggctgaac actcccgatt aaggattctg tgctttagac 1860

tttggctgtt gtgctaaagg actgagtata ggtggaggtt ttcagacctt ggaagaaggt 1920

cccacggtga acttgtgctg tgaacttgca cacttggggc a 1961

<210> 3
<211> 2839
<212> DNA
<213> Mus musculus

<300>
<302> Interspecies Diversity of the Occludin Sequence: cDNA Cloning of Human, Mouse, Dog, and Rat-Kangaroo Homologues
<303> J. Cell Biol.
<304> 133
<305> 1
<306> 43-47
<307> April 1996
<308> U41984, U49221, U41985

<400> 3

ggagtttcag gtgaatgggt caccgaggga ggaggctggc cacgccacac ctcgtcgcta 60

gtgcccacct cccggcccct ctttccttag gcgacagcgg tggagttgcg ggagagcggt 120

ccagcgcacg gagcaaccgg ctaggggctc ggcaggttcg cttatcttgg gagcctggac 180

attttgctca tcataaagat taggtgacca gtgacatcag ccatgtccgt gaggcctttt 240

gaaagtccac ctccttacag acctgatgaa ttcaaaccca atcattatgc accaagcaat 300

gacatgtatg gcggagagat gcatgtccgg ccgatgctct ctcagccagc gtactctttt 360

tatccggaag atgaaattct tcacttctac aaatggacgt cgcccccagg ggtgatccgg 420

atcctgtcta tgctcattat tgtgatgtgc atcgccatat ttgcctgtgt ggcttccaca 480

cttgcttggg acagaggcta tgggacaggg ctctttggag gaagcctaaa ctacccttat 540

agtggctttg gctacggagg tggctatgga ggcggctatg gaggctatgg ctatggctat 600

ggcggatata cagacccaag agcagccaaa ggcttcctgt tggccatggc agccttctgc 660

ttcatcgctt ccttagtaat atttgtgacc agtgttataa gatctggaat gtccaggaca 720

agaagatatt acttgatcgt gatcatagtc agcgctatcc tgggcatcat ggtgtttatt 780

gccacgatcg tgtacataat gggagtgaac ccgacggccc aggcttctgg atctatgtac 840

ggctcacaga tatatatgat ctgcaaccag ttttatactc ctggaggtac tggtctctac 900

gtggatcaat atttgtatca ctactgtgtg gttgatcccc aggaggctat agccattgtc 960

ctgggggttca tgattatcgt ggcttttgct ttaatcatct tttttgctgt gaaaacccga 1020

agaaagatgg atcggtatga taagtccaat attttgtggg ataaggaaca catttatgat 1080

gaacagcccc ccaatgttga agagtgggtt aaaaatgtgt ctgcaggcac acaggacatg 1140

cctccacccc catctgacta tgcggaaaga gttgacagtc caatggccta ctcctccaat 1200

ggcaaagtga atggcaagcg atcataccca gagtctttct ataagtcaac acctctggtg 1260

cctgaagtgg cccaggagat tcctctgacc ttgagtgtgg atgacttcag gcagcctcgg 1320

tacagcagca atggtaacct agagacacct tctaaaaggg ctcccacgaa ggggaaagca 1380

ggaaagggca agaggacgga ccctgaccac tatgaaacag actacacgac aggtggggag 1440

tcctgcgagg agctggagga ggactgggtc agggaatatc cacctatcac ttcagatcaa 1500

caaagacaac tctacaagag aaattttgat gcaggtctgc aggagtataa gagcttacag 1560

gcagaactag acgacgtcaa taaagagctc tctcgtctag ataaagagct ggatgactac 1620

agagaggaga gtgaagagta catggctgct gctgatgaat ataatagact aaagcaagtt 1680

aagggatctg cagattataa aagtaagagg aattactgca agcagttgaa gagcaaatta 1740

tcgcacatca agaggatggt gggagactat gacagacgga aaccttagag agatgccagt 1800

tgcgggagaa gggagaggtg catctgcctg cacgatgtct ctgcaattct ctccagaggc 1860

aaactgactt tggactctaa tctgggaagt taaaactttg tgatcattac aaagtttcca 1920

tggctttaat tccatcagtt tcctatctcc agtattgaag cattttataa atggcttttg 1980

ataattgact gggctgaaca ctccaattaa ggattttaca gtttcaacat tgattcttgt 2040

attaagaatt aaaatgttgc ttgaggtttt aaatgtcaag aaaggtcctg gtgtgagctg 2100

tgatgtgtgt gagctgtgat gtgaaggttc acacgccagg cagcgtgttc ctccaggtag 2160

accgtctaat caatctttgc agcagccctc aggtgactgt tatttagaat caggttgttt 2220

ttggttttcc agacagggtt tctctgtgta gccctggctg acctagaact tacgctgtag 2280

accaggctgg ccttgaactc acacagctcc tctgagtgct ggtgcaggag ttaacgtcgt 2340

ggaccggtat catcactttt cctgcggtga cttctccaaa ctgaaactgc taaggcagtt 2400

ttggctaagt ctgttttatg actgcaaatg acagcattcc tgcctttgta tttcagggga 2460

aatacgatac attatatcgg ccatgttccc caccactgtt tttcttatat tgacttttaa 2520

caaatgaata ggattatttt tggctttaca ttttttccta acacttaaga tcatataaaa 2580

ttaacaaata tgtgaaattt aagaattgta aatatatatt tacgtttgaa agatgatttt 2640

aaatccaggg ttaaagtgct ttttatcttg tatagtttac atgctttttt ttttttttga 2700

taacccacta gacctttcca ttgtatcaga gtatccaatt acatttacaa ttatgacttg 2760

aattgtattt cacaggaatg ctcaagtttt gtacatattt tataaggtat taaacctgat 2820

gttctctttc taaaaaaaa 2839

<210> 4
<211> 1920
<212> DNA
<213> Gallus gallus

<300>
<302> Occludin: A novel integral membrane protein localizing at tight junctions
<303> J. Cell Biol.
<304> 123
<305> 6
<306> 1777-1788
<307> Dec 1993
<308> D21837
<313> 1 TO 1920

<400> 4

caaagcggcg gagggccacc atgttcagca agaagtccta cgacggcccc cccgcggggt 60

acggcccccc cacggggtac ggcgccccca cggctgatta cggctacggg tctccgccgc 120

cgggctccta ctacgtggac gacgctccgc agctcttcta caagtggacg tcgccgcccg 180

gcgcggtgcg ggggctgcag gcgggggtcc tcgtgctgtg catcgccatc ttcgcctgcg 240

tcgcttccac gctcgcctgg gattacggct acggcctggg gggggcgtac ggcaccgggc 300

tgggggggtt ctacggctcc aactactacg gcagcgggct gagctacagc tacggctacg 360

ggggctacta cggaggggtg aaccagcgca cggccaacgg cttcatgatc gccatggccg 420

tgctgtgctt cctggcccag ctggggctgc tggtggcggc gctcagcaaa tccggggcca 480

cgcgctcgcg gcgcttctac ctggccgtgc tggtgctgag cgccgtgctg gccttcgtca 540

tgctcatcgc ctccatcgtc tacatcatgg gcgtcaaccc gcaggcgcag atgtccagcg 600

gttactacta cagccccttg ttggccatgt gcagccaggc ctacggcagc acctacctca 660

accagtacat ctaccactac tgcaccgtgg acccccagga ggctgtggct gctgtctgtg 720

ggttcctcat cgtcatcctg ctctgcctca tctgcttctt cgcccagaag acgcgcagta 780

agatctggcg ctacggcaaa gccaacatct actgggaccg cgcgcccgtg gtgcaggagg 840

ggcctgacgt ggaggagtgg gtgaagaacg tggcggatgg ggccagcgtg caggacgaga 900

cggccacgct tgcctactcg gagaagccca ccagccctgt cgccgccccc ccctacagct 960

acgtgccccc ccccagcgct gggtactacc cctcgggcac ctacagcagc cggggcgacc 1020

agccggaccg ggccctcagt gccagccctg tgcatgggga ggaggaggag gagaagggga 1080

aggatcagcc cagcagaccg cccgcccgcc ggggccgccg ccgccgccgt aacccccgagt 1140

tggatgagtc ccagtatgag accgactaca ccacggccgt ggagtccagt gatgagcggg 1200

accaggagca gtgggccagt ctgtaccccc ccatcacgtc ggacggcgcc cgccagcgct 1260

acaagcagga gttcgacacc gacctgaagc gctacaagca gctctgtgct gagatggaca 1320

gcatcaacga ccgcctcaat cagctcagcc gacggctcga cagcatcacc gaggacagcc 1380

ctcaatacca ggatgtggca gaggagtaca atcagctcaa agacctgaag cggagcccag 1440

actaccaaag caagaagcag gagagcaaag tgctgcgcaa caagctcttc cacatcaagc 1500

gcatggtgag cgcctacgac aaggtgcggg ggtaaccagc accagcagga gggggggca 1560

cccccacccc cccacccaaa gactgttgtg cattttgtac cttcttctta aaagaaaaaa 1620

cacactcaat cacatccacc cccccccaa aggggtcacc ccccccaacc cccccttaac 1680

ccctcccact ccatccactg tgaatgcacc cactgagtgt tgggggctgc cccccccccc 1740

cgtgctgtta ttatggtagt gggggggggg ggggagctgt gtaaggccca cacctctggg 1800

aagggctgag gtctgcaaca gcatcacagc cgccctggca ctgtgggtac cagaatggta 1860

ccctgagcac acagctcaag gtgggggggg gagcatccct tgggatgggg gggggggca 1920

<210> 5
<211> 17
<212> DNA
<213> Artificial Sequence

<220>
<223> Description of Artificial Sequence: Human occludin antisense oligonucleotide

<400> 5
aagaggcctg gatgaca 17

<210> 6
<211> 17
<212> DNA
<213> Artificial Sequence

<220>
<223> Description of Artificial Sequence: Human occludin scrambled oligonucleotide

<400> 6
gcaagtcagg acgtaga 17